# FASCICULE DE BREVET EUROPEEN

(11) **EP 0 870 756 B1**
(45) Date de publication et mention de la délivrance du brevet: **07.11.2001**
(21) Numéro de dépôt: 98400820.1
(22) Date de dépôt: 06.04.1998
(51) Int. Cl.: C07C 209/18

(54) **Procédé de préparation des dinitroanilines 3,4-disubstituées**
Verfahren zur Herstellung von 3,4 disubstituirten Dinitroanilinen
Process for the preparation of 3,4-disubstituted dinitroanilines

(30) Priorité: 07.04.1997 FR 9704235; 02.07.1997 FR 9708359
(43) Date de publication de la demande: 14.10.1998
(73) Titulaire: CFPI NUFARM, 92233 Gennevilliers (FR)
(72) Inventeur: Schapira, Joseph, 75015 Paris (FR); Cheminaud, Jean-Claude, 95220 Herblay (FR); Gasse, Jean-Jacques, 27600 Gaillon (FR); Schanen, Vincent, 75019 Paris (FR); Rondot, Benoit, 92300 Levallois Perret (FR); Lemoine, Jean-Claude, 92290 Chatenay Malabry (FR)
(74) Mandataire: Koch, Gustave

(56) Documents cités:
- EP-A- 0 630 883
- WO-A-97/01527

## Description

L'invention a pour objet un procédé de préparation des dinitroanilines 3,4-disubstituées.

Les dinitroanilines en question, qui sont des herbicides connus, agissent essentiellement comme inhibiteurs de la division cellulaire.

Elles sont représentées par la formule 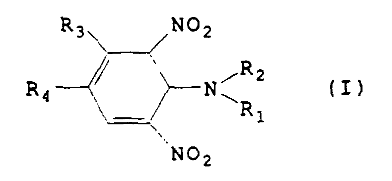 dans laquelle
- R₁ et R₂, qui sont identiques ou différents l'un de l'autre, représentent un atome d'hydrogène, un radical alkyle rectiligne ou ramifié saturé en C₁ à C₆, un radical alkylène linéaire ou ramifié en C₂ à C₆, un radical cyclopropyle ou un radical chloroéthyle,
- R₃ et R₄, qui peuvent être identiques ou différents l'un de l'autre, sont choisis dans le groupe comprenant l'atome de chlore, le groupe amino, les radicaux alkyle inférieurs en C₁ à C₃ et le radical trifluorométhyle.

Des dinitroanilines 3,4-disubstituées particulièrement intéressantes sont la pendiméthaline, la prodiamine et la dinitramine respectivement représentées par les formules:

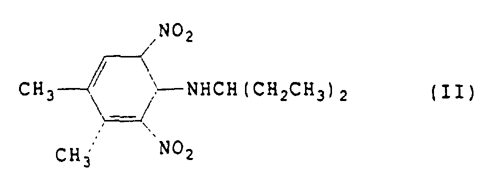

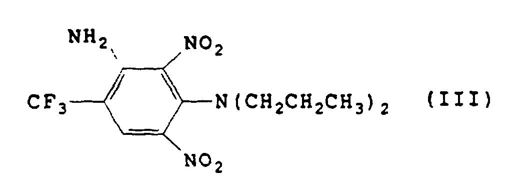

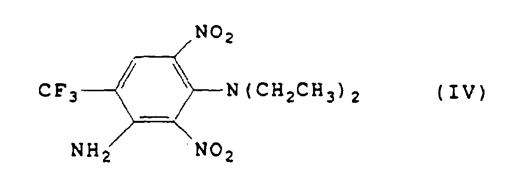

Il a déjà été proposé, par le brevet US-A-5 475 148, de préparer la pendiméthaline et plus généralement les N-alkyl-3,4-dialkyl-2,6-dinitroanilines en procédant successivement:
- à la dinitration du 3,4-dialkylphénol en utilisant l'acide nitrique en milieu diphasé,
- à l'alkylation du 2,6-dinitro-3,4-dialkylphénol obtenu à l'étape précédente préférentiellement en présence d'un catalyseur de transfert de phase,
- à la réaction du 3,4-dialkyl-2,6-dinitro-alcoxybenzène formé à l'étape précédente avec une amine en présence d'une quantité catalytique de base ou d'halogénure.

Ce procédé n'est pas compétitif, notamment en raison du très mauvais rendement de l'étape de dinitration.

De plus, il est limité à la préparation des dinitroanilines 3,4-dialkyl-substituées.

L'invention a pour but, surtout, de remédier à cet inconvénient et de fournir un procédé de préparation des dinitroanilines disubstituées en position 3 et 4 de façon identique ou différente par des radicaux alkyle inférieurs en C₁ à C₃, le radical trifluorométhyle, le groupe amino ou l'atome de chlore, dont le rendement soit compatible avec les desiderata de la pratique.

Et la Société Demanderesse a le mérite d'avoir trouvé, à l'issue de recherches approfondies, que ce but pouvait être atteint par la mise en oeuvre d'un procédé comprenant successivement une étape de dinitration d'un phénol 3,4-disubstitué, une étape d'alkylation du dérivé dinitré ainsi obtenu et une étape d'amination du 2,6-dinitro-alcoxybenzène 3,4-disubstitué ainsi obtenu et
caractérisé par le fait que:
- l'étape de dinitration du phénol 3,4-disubstitué correspondant à la dinitroaniline 3,4-disubstituée recherchée est effectuée au sein d'un milieu réactionnel comprenant un faible excès d'agent nitrant, une quantité suffisante de protons et un catalyseur choisi dans le groupe constitué par les sels solubles des métaux de transition des colonnes IV à XII de la classification périodique, de préférence les sels solubles des ions Fe_{III}, Fe_{II}, Zn_{II} et Cu_{II}, et que
- une étape d'O-alkylation du phénol 3,4-disubstitué dinitré obtenu à l'étape précédente, est effectuée à l'aide d'un agent alkylant choisi dans le groupe comprenant, d'une part, les mono-halogénures d'alkyle linéaires ou ramifiés ayant au moins 3 atomes de carbone et pouvant comprendre un cycle saturé ou au moins une insaturation, d'autre part, les polyhalogénures d'alkyle linéaires ou ramifiés ayant au moins 2 atomes de carbone et pouvant comprendre un cycle saturé ou au moins une insaturation, d'autre part encore les polyalcoxy-haloalkyléthers représentés par la formule (RO)ₙR'X dans laquelle X représente un atome d'hydrogène, de chlore, de brome ou d'iode et R et R', indépendamment l'un de l'autre, un radical méthyle, éthyle ou propyle, n étant tel que le nombre d'atomes de carbone de l'agent alkylant est ≥ 2, notamment les chlorométhyl-polyméthoxy éthers et les chlorométhyl-polyéthoxy éthers, les oxydes d'éthylène et de propylène et, d'autre part encore, les composés de structure répondant à la formule 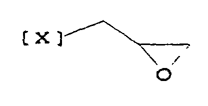 qui représente des épihalohydrines lorsque X représente Cl ou Br, le glycidol lorsque X représente OH, les éthers de glycidyle lorsque X représente OR, R étant une chaîne aliphatique d'au plus 12 atomes de carbone, ou un oxiranne lorsque X est une chaîne aliphatique R de 1 à 12 atomes de carbone.

Selon un mode de réalisation avantageux du susdit procédé, le catalyseur mis en oeuvre pendant l'étape de dinitration est choisi dans le groupe comprenant les chlorures ferriques et ferreux, les chlorures de zinc et cuivrique, les nitrates ferriques, zinciques et cuivriques ainsi que les sulfates ferriques, ferreux, zinciques et cuivriques.

La proportion de catalyseur par rapport au phénol est de 1 à 50‰, de préférence de 5 à 30‰.

L'agent nitrant est choisi dans le groupe comprenant l'acide nitrique et les nitrates alcalins et alcalino-terreux solubles dans l'eau.

L'excès d'agent nitrant mis en oeuvre pour l'étape de dinitration est de 10% à 30% en poids.

La quantité de protons qui doit être présente dans le milieu nitrant est de 2 à 7, de préférence de 2,4 à 6 et, plus préférentiellement encore, de 2,6 à 2,9 équivalents par rapport au phénol et peut être apportée par un acide appelé co-acide et constitué par un acide minéral choisi dans le groupe comprenant les acides chlorhydrique, bromhydrique, sulfurique et phosphorique, ou par un acide organique choisi dans le groupe comprenant les acides carboxyliques inférieurs dont notamment les acides acétique, propionique et oxalique.

L'étape de nitration peut être réalisée en milieu nitrique homogène ou en présence d'un solvant inerte.

Le solvant inerte est avantageusement choisi dans le groupe comprenant les essences aliphatiques dont notamment l'hexane, l'heptane, l'octane, le nonane et le dodécane, les dérivés chlorés dont notamment le dichlorométhane, le 1,2-dichloroéthane, le chloroforme, le trichloréthylène, le tétrachloréthylène, et les dialkyl-éthers dont notamment les diéthyl- et dipropyl-éthers ainsi que les dibutyléthers.

La réaction est avantageusement conduite sous pression atmosphérique et à des températures de -20°C à +80°C.

Le dérivé 2,6-dinitré obtenu peut être séparé et purifié par les techniques conventionnelles bien connues de l'homme du métier.

Lorsque l'étape de nitration est effectuée sans solvant, le dérivé dinitré est précipité par dilution et glaçage du milieu réactionnel, puis séparé par filtration.

Au contraire, lorsque l'étape de nitration est effectuée au sein d'un solvant, le dérivé dinitré solubilisé dans la phase organique est isolé par décantation, l'agent nitrant contenu dans la phase aqueuse pouvant alors être recyclée au stade de la nitration après avoir été soumis au préalable à une concentration.

La pureté du dérivé 2,6-dinitrophénol 4-substitué obtenu est généralement excellente et ne nécessite pas de purification ultérieure.

Lorsque le solvant est judicieusement choisi, il ne devient pas nécessaire d'isoler le dinitrophénol et la solution solvantée peut alors directement être soumise à l'étape suivante; c'est le cas par exemple lorsque le solvant est un solvant halogéné choisi dans le groupe comprenant les chlorures de méthylène, le 1,2-dichloroéthane, le 1,2-dibromoéthane, le chloropropane, le chlorure d'isopropyle, le bromure d'isopropyle, les chlorures de butyle, les 1,2-dichloropropane et 1,2-dibromopropane.

Le rendement de l'étape de dinitration est d'au moins 92% et peut atteindre 99%.

Selon un autre mode de réalisation avantageux du procédé conforme à l'invention, l'agent alkylant mis en oeuvre pendant l'étape d'alkylation est soit un monohalogénure d'alkyle représenté par la formule CₙH₂ₙ₋₁X dans laquelle X est un atome de chlore, brome ou iode et n est ≥ 3, soit un polyhalogénure d'alkyle représenté par la formule CₙH₂ₙ₋ₘX₂₊ₘ dans laquelle X est un atome de chlore, brome ou iode et n ≥ 2 avec O ≤ m ≤ 2n.

L'agent alkylant peut être choisi dans le groupe comprenant,
- en tant que monohalogénures d'alkyle, les halogénures de n-propyle, d'isopropyle, de n-butyle, de sec-butyle, d'isobutyle, de tertbutyle, de n-pentyle, de sec-amyle, de 3-pentyle, de neopentyle, d'hexyle, d'isohexyle, de cyclohexyle, de cyclopropyle, de benzyle, d'allyle, et de polyalcoxyalkyle avec comme chaîne alcoxylée un radical méthoxy, éthoxy ou propanoxy,
- en tant que polyhalogénures d'alkyle, les dihaloéthanes, notamment le dichloroéthane et le dibromo-éthane, les dihalopropanes, notamment les 1,2- et 1,3-dichloropropanes.

Les conditions des réactions d'éthérification des dinitrophénols sont bien connues de l'homme du métier.

Généralement, ces réactions sont effectuées sur le phénol dinitré ou sur un phénate alcalin correspondant en milieu aqueux ou plus avantageusement en milieu biphasique par réaction de transfert de phase.

Le phénate alcalin peut être un dinitrophénate soit de sodium ou de potassium, soit d'un sel organique tel que l'ammonium, les tétralkyl-ammonium quaternaires et les tétralkylphosphonium quaternaires.

L'agent d'alkylation, en solution dans un solvant immiscible à l'eau, est mélangé avec une solution aqueuse du phénate alcalin en présence d'un catalyseur de transfert de phase qui peut être constitué par un halogénure d'ammonium ou un sel de phosphonium quaternaire.

Le solvant peut être choisi dans le groupe comprenant les solvants aromatiques, les essences aliphatiques et les cétones; de préférence, il est choisi dans le groupe comprenant le toluène, l'O-xylène, le m-xylène, le p-xylène, l'hexane, l'heptane, l'octane, le nonane, le dodécane, la méthyléthylcétone, la méthyliso-butylcétone, la cyclohexanone et les mélanges de ces solvants.

Il est également possible d'utiliser comme solvant un excès d'agent alkylant.

Les catalyseurs préférés sont le chlorure de tétra-butyl-ammonium, le bromure de tétrabutyl-ammonium, le bromure de tétrabutyl-phosphonium, le chlorure de triéthyl-benzyl-ammonium, le triméthylbenzyl-ammonium.

La réaction d'alkylation peut être conduite à une température de 15°C à 150°C, sous pression atmosphérique ou sous une pression pouvant aller jusqu'à 15 bars.

Les réactions par transfert de phase peuvent être pratiquées à des températures variant de -10°C à +50°C et à pression atmosphérique alors que les réactions mettant en oeuvre des halogénures d'alkyle inférieurs peuvent se faire sous une pression de plusieurs dizaines de bars.

Le phénoxyéther dinitré obtenu à l'issue de cette étape peut être isolé du milieu réactionnel par les techniques connues de l'homme du métier, telles que par exemple l'extraction par un solvant approprié ou encore la concentration suivie d'un fractionnement de la phase organique lorsque le procédé met en oeuvre une réaction de transfert de phase.

Lorsqu'un excès d'agent alkylant est utilisé comme solvant, le produit est isolé par solubilisation des sels solubles dans l'eau, un second lavage alcalin permettant de solubiliser le dinitrophénate qui n'a pas réagi; une contre-extraction en milieu acide du produit résultant de ce second lavage conduit alors à une solution de dinitrophénol qui peut être facilement recyclée dans une opération subséquente d'éthérification.

La phase organique isolée après le second lavage est soumise à une évaporation de l'excès d'agent alkylant qui peut être avantageusement réutilisé dans l'opération suivante.

Toutefois, lorsque le solvant est choisi dans le groupe comprenant le toluène, le xylène, les essences aliphatiques tels que les heptane, octane, nonane, décane et dodécane, le fractionnement n'est pas nécessaire et la solution organique obtenue à cette étape peut être soumise directement à l'étape d'amination.

L'étape d'amination est réalisée en faisant réagir, sous pression atmosphérique à la température de reflux du milieu, le produit de l'étape d'alkylation avec un excès d'amine secondaire ou primaire choisie dans le groupe comprenant l'amino-3-pentane, la N,N-diéthylamine, la N,N-dipropylamine, la N-éthyl-,N-butylamine, la N-propyl, N-méthyl-cyclopropylamine, la N-(2-chloroéthyl), N-propyl-amine, la N-propyl, N-2-méthyl-2-propénylamine, la N-éthyl, N-2-méthyl-2-propénylamine au sein d'un solvant inerte choisi dans le groupe comprenant les alcools et les solvants aromatiques.

De préférence, on utilise un excès d'amine pouvant dépasser 9 moles d'amine pour 1 mole du produit obtenu à l'étape d'alkylation.

De façon tout à fait surprenante, le procédé conforme à l'invention permet d'atteindre des rendements très élevés, supérieurs à 96%, sans production de plus de 1 ppm de N-nitrosamines et sans risque de formation de produits détonants.

Les étapes successives du procédé selon l'invention apparaissent sur le schéma réactionnel suivant:

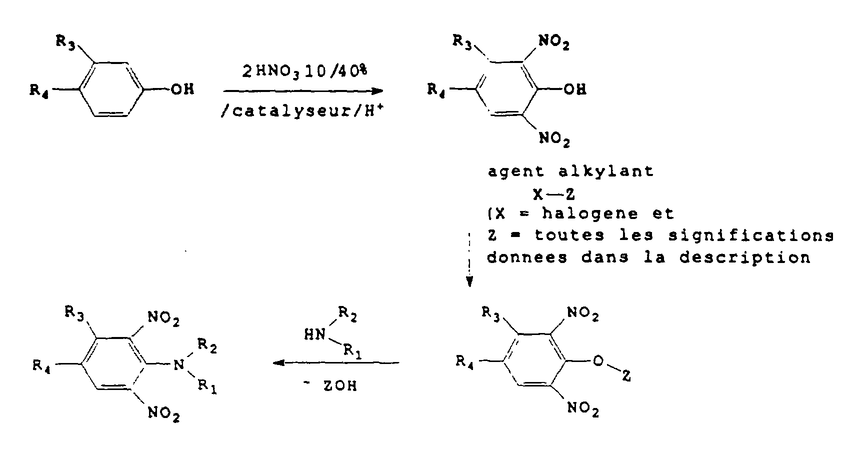

Lorsque l'agent alkylant est polyfonctionnel, c'est-à-dire constitué par exemple par un dihaloéthane, il est susceptible de réagir avec deux molécules de dinitrophénol et il se produit une polyéthérification illustrée par le schéma réactionnel:

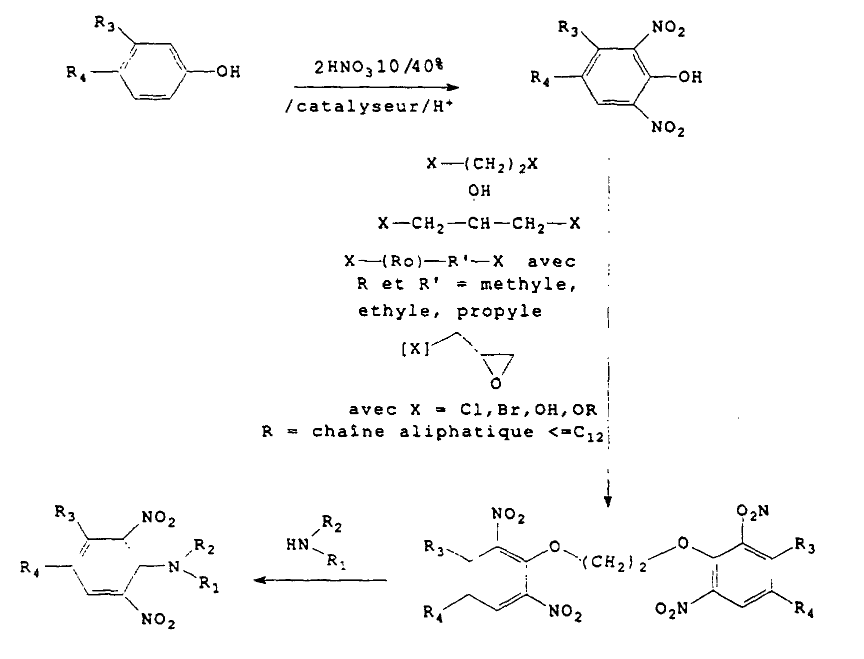

L'invention pourra être bien comprise à l'aide des exemples non limitatifs qui suivent et dont certains sont relatifs à des modes de réalisation avantageux.

Les exemples 1 à 9 illustrent l'étape de dinitration du phénol 3,4-disubstitué.

### EXEMPLE 1

Cet exemple illustre l'étape de dinitration du phénol 3,4-disubstitué.

On introduit en l'espace de 20 minutes et en maintenant la température à 20°C, une solution de 4,5 g (35,7 mmol) de 3,4-diméthyl-phénol dans 40 g de 1,2-dichloroéthane dans un milieu nitrant constitué d'un mélange de 23,1 g (93 mmol) d'acide nitrique à 25,5% et de 7,0 g d'acide chlorhydrique à 37% (71 mmol); ce milieu nitrant contient par ailleurs 80 mg de chlorure zincique.

L'excès d'agent nitrant est de 30% et la quantité de protons présente correspond à 164 mmol, soit 4,6 équivalents par rapport au phénol.

On laisse réagir pendant deux heures et demi à la température de 50°C.

On sépare les phases par décantation; la phase aqueuse est extraite deux fois avec 20 g de dichloroéthane et les phases organiques sont regroupées, séchées sur sulfate de magnésium et évaporées à sec.

On recueille 6,88 g d'un solide jaune-orangé comportant 98,8% de 2,6-dinitro-3,4-diméthyl-phénol, ce qui correspond à un rendement de 90,9%.

### EXEMPLE 2

Cet exemple illustre l'étape d'alkylation du dinitrophénol 3,4-disubstitué.

Dans un ballon de 50 ml équipé d'un agitateur, d'un réfrigérant et d'un thermomètre, on charge 5 g de 2,6-dinitro-3,4-diméthyl-phénate de tétrabutylammonium et 50 g de 1,2-dichloroéthane.

On porte le mélange au reflux pendant 11 heures.

On le refroidit ensuite à 25°C, puis on le lave successivement avec 20 g d'HCl 0,1N, puis trois fois avec 17 g de soude 0,1M, puis avec 30 ml d'HCl 0,1N et, enfin, avec 35 g d'eau distillée.

On récupère une phase organique qui est séchée sur sulfate de magnésium puis évaporée sous vide.

On recueille 2,65 g d'un solide jaune-orangé comportant 99% (détermination par chromatographie en phase gazeuse) de 2-(2,6-dinitro-3,4-diméthyl-phénoxy)-1-chloro-éthane.

Les phases aqueuses mères et de lavage sont contre-extraites à pH 1 avec 130 g de 1,2-dichloroéthane.

Après séchage et évaporation du solvant, on recueille 0,15 g d'un solide orange comprenant 61,8% de 2,6-dinitro-3,4-diméthylphénol, 18,1% de 2-(2,6-dinitro-3,4-diméthylphénoxy)-1-chloroéthane et 16,7% d'hydroxyde de tétrabutylammonium, ce produit pouvant être recyclé en l'état dans une opération ultérieure.

### EXEMPLE 3

Cet exemple illustre l'étape d'alkylation du dinitrophénol 3,4-disubstitué.

Dans un ballon de 50 ml équipé d'un agitateur, d'un réfrigérant et d'un thermomètre, on charge 5 g de 2,6-dinitro-3,4-diméthyl-phénate de tétrabutylammonium obtenu par salification de 2,6-dinitro-3,4-diméthyl-phénol, puis 1,78 g de bromure de benzyle (soit 0,945 équivalent) et 10 g de toluène.

On porte à 55 ±2°C durant 12 heures, puis pendant 4 heures à 65°C.

L'avancement de la réaction est suivi par chromatographie sur couche mince.

Après disparition totale du phénol, le milieu réactionnel est concentré sous vide; le résidu est ensuite repris par 70 g de 1,2-dichloroéthane, puis lavé successivement deux fois avec 35 g d'HCl 1N.

La phase organique séparée par décantation est lavée trois fois avec 30 g de soude à 0,1M, puis avec 25 g d'HCl 1N, puis de nouveau avec 35 g de soude à 0,1M, puis avec 35 g de soude 1M, et enfin avec 30 g d'eau distillée.

La phase organique recueillie est séchée sur sulfate de magnésium puis évaporée sous vide.

On recueille 2,53 g d'un solide jaune-orangé constitué à 94% (déterminé par chromatographie en phase gazeuse ou CPG) de 2,6-dinitro-3,4-diméthyl-phényl-benzyléther avec un rendement de 80%.

### EXEMPLE 4

Cet exemple illustre l'étape d'amination.

Dans un tricol de 25 ml équipé d'un agitateur, d'un réfrigérant et d'un thermomètre, on charge 2 g de 2-(2,6-dinitro-3,4-diméthyl-phénoxy)1-chloroéthane à 99% de pureté, 6,1 g d'amino-3-pentane et 3,1 g de méthanol.

On maintient le mélange à 45°C pendant 8 heures, puis à 55°C pendant 9 heures.

La chromatographie sur couche mince d'une partie aliquote de milieu réactionnel annonce la fin de la réaction par disparition de l'éther.

On évaporé ensuite le solvant et l'excès d'amine sous vide.

Le résidu obtenu est repris au dichlorométhane puis lavé avec 50 g d'HCl 0,1N, puis avec 20 g de soude 1M et, enfin, avec 30 g d'eau jusqu'à neutralité; il est ensuite séché puis évaporé à sec sous vide.

On obtient 2,02 g d'un solide jaune-orangé comprenant 99,1% (déterminé par chromatographie en phase gazeuse ou CPG) de 3,4-diméthyl-N(1-éthyl-propyl)-2,6-dinitrobenzénamine.

### EXEMPLE 5

Cet exemple illustre l'étape d'amination.

Dans un tricol de 25 ml équipé d'un agitateur, d'un réfrigérant et d'un thermomètre, on charge 2 g de 2,6-dinitro-3,4-diméthyl-phényl-benzyléther à 94% de pureté, 8,3 g d'amino-3-pentane et 4 g d'isopropanol.

On maintient le mélange à 55°C pendant 9 heures, puis à 75°C pendant 8 heures.

La chromatographie sur couche mince d'une partie aliquote de milieu réactionnel annonce la fin de la réaction par disparition de l'éther.

On évapore ensuite le solvant et l'excès d'amine sous vide.

Le résidu obtenu est repris à l'isopropyléther; la suspension obtenue est filtrée et le filtrat est lavé avec 50 g de soude 1M, puis avec 20 g d'eau jusqu'à neutralité avant d'être séché puis évaporé à sec à 95°C sous un vide de 7 mm Hg.

On obtient 1,0 g d'un mélange de 3,4-diméthyl-N(1-éthyl-propyl)-2,6-dinitrobenzénamine et de 3,4-diméthyl-2,6-dinitrophénol que l'on sépare par chromatographie sur couche mince.

La quantité de 3,4-diméthyl-N(1-éthyl-propyl)-2,6-dinitrobenzénamine ainsi recueillie est de 0,4 g.

### EXEMPLE 6

Cet exemple illustre l'étape de O-alkylation par un oxiranne d'un dinitrophénol 3,4-disubstitué.

Dans un réacteur de 100 ml à agitation magnétique muni d'un manomètre et d'une pastille de sécurité (40 bars), on charge 20 g de 2,6-dinitro-3,4-diméthylphénol à 98,6% de pureté, 10 g de toluène et 0,2 g de pyridine. On refroidit à 10°C et on coule 6,54 g d'oxyde de propylène. On ferme le réacteur et on met sous 2-3 bars d'azote.

On porte à 120°C pendant 3 heures.

On refroidit à 30°C et on décompresse.

Après évaporation du toluène, on recueille 26,4 g d'une huile marron composée de 94,01% de 2-propanol-1-(2,6-dinitro-3,4-diméthylphénoxy) et de 1,51% de (2,6-dinitro-3,4-diméthylphénoxy)-propanoxy-propanol.

### EXEMPLE 7

Cet exemple illustre l'étape de O-alkylation par un oxiranne d'un dinitrophénol 3,4-disubstitué.

Dans un réacteur de 100 ml à agitation magnétique muni d'un manomètre et d'une pastille de sécurité (40 bars), on charge 20 g de 2,6-dinitro-3,4-diméthylphénol à 98,06% de pureté, 10 g de xylène et 0,1 g de pyridine. On refroidit à 10°C et on coule 5 g d'oxyde d'éthylène. On ferme le réacteur et on met sous 2-3 bars d'azote.

On porte à 120°C pendant 7 heures.

On refroidit à 30°C et on décompresse.

Après évaporation du xylène, on recueille 24,07 g de solide marron composé de 92,8% d'éthanol-2-(2,6-dinitro-3,4-diméthylphénoxy) et de 1,73% de (2,6-dinitro-3,4-diméthyl-phénoxy) -éthoxy-éthanol.

### EXEMPLE 8

Cet exemple illustre l'étape d'amination du 1-(2,6-dinitro-3,4-diméthylphénoxy)2-propanol.

Dans un tricol de 100 ml équipé d'un agitateur magnétique, d'un réfrigérant (-10°C) et d'un thermomètre, on charge 40 g de 2-propanol-1-(2,6-dinitro-3,4-diméthyl-phénoxy) à 96,2% de pureté et à 2,53% de produit de diaddition, 141,4 g d'amino-3-pentane à 44,7% dans l'eau et 0,8 g de CaCl₂,6H₂0.

On maintient le mélange à reflux (83°C) pendant 8 heures.

En pratiquant une chromatographie sur couche mince d'une partie aliquote de milieu réactionnel, on détecte la fin de la réaction par disparition de l'éther.

On distille l'azéotrope eau/amine en excès; le résidu à 80°C est lavé avec 100 g d'eau saturée à NaCl, puis repris au dichlorométhane; il est ensuite évaporé sous vide.

On obtient 37,15 g de solide orange constitué à 92,38% (déterminé par CPG) de N-(1-éthylpropyl)-2,6-dinitro-3,4-diméthyl-benzènamine.

### EXEMPLE 9

Cet exemple illustre l'étape d'amination du 1-(2,6-dinitro-3,4-diméthylphénoxy)2-éthanol.

Dans un tricol de 100 ml équipé d'un agitateur magnétique, d'un réfrigérant (-10°C) et d'un thermomètre, on charge 24 g d'éthanol-2-(2,6-dinitro-3,4-diméthylphénoxy) à 92,8% de pureté et à 1,73% de (2,6-dinitro-3,4-diméthylphénoxy)éthoxy-éthanol, 65,03 g d'amino-3-pentane à 60% dans l'eau et 0,5 g de CaCl₂,6H₂0.

On maintient le mélange à reflux (84°C) pendant 7 heures.

En pratiquant une chromatographie sur couche mince d'une partie aliquote de milieu réactionnel, on détecte la fin de la réaction par disparition de l'éther.

On distille l'azéotrope eau/amine en excès; le résidu obtenu est repris au xylène puis lavé avec 30 g de HCl à 5%, puis 40 g de soude à 5% et enfin avec 100 g d'eau jusqu'à neutralité; il est ensuite évaporé sous vide.

On obtient 23,4 g de solide orange constitué à 92,31% (déterminé par CPG) de N-(1-éthylpropyl)-2,6-dinitro-3,4-diméthyl-benzénamine.

## Revendications

1. Procédé de préparation des dinitroanilines 3,4-disubstituées de formule 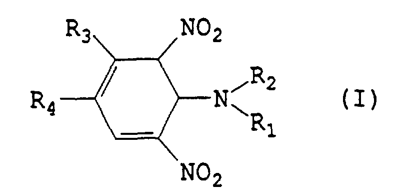 dans laquelle
- R₁ et R₂, qui sont identiques ou différents l'un de l'autre, représentent un atome d'hydrogène, un radical alkyle rectiligne ou ramifié saturé en C₁ à C₆, un radical alkylène linéaire ou ramifié en C₂ à C₆, un radical cyclopropyle ou un radical chloroéthyle,
- R₃ et R₄, qui peuvent être identiques ou différents l'un de l'autre, sont choisis dans le groupe comprenant l'atome de chlore, le groupe amino, les radicaux alkyle inférieurs en C₁ à C₃ et le radical trifluorométhyle, comprenant successivement une étape de dinitration d'un phénol 3,4-disubstitué, une étape d'alkylation du dérivé dinitré ainsi obtenu et une étape d'amination du 2,6-dinitro-alcoxybenzène 3,4-disubstitué ainsi obtenu et
**caractérisé par le fait que**:
- l'étape de dinitration du phénol 3,4-disubstitué correspondant à la dinitroaniline 3,4-disubstituée recherchée est effectuée au sein d'un milieu réactionnel comprenant un excès d'agent nitrant de 10 à 30% en poids, une quantité de protons de 2 à 7 équivalents par rapport au phénol et un catalyseur choisi dans le groupe constitué par les sels solubles des métaux de transition des colonnes IV à XII de la classification périodique, de préférence les sels solubles des ions Fe_{III}, Fe_{II}, Zn_{II} et Cu_{II}, et que
- une étape d'O-alkylation du phénol 3,4-disubstitué dinitré obtenu à l'étape précédente, est effectuée à l'aide d'un agent alkylant choisi dans le groupe comprenant, d'une part, les mono-halogénures d'alkyle linéaires ou ramifiés ayant au moins 3 atomes de carbone et pouvant comprendre un cycle saturé ou au moins une insaturation, d'autre part, les polyhalogénures d'alkyle linéaires ou ramifiés ayant au moins 2 atomes de carbone et pouvant comprendre un cycle saturé ou au moins une insaturation, d'autre part encore les polyalcoxy-haloalkyléthers représentés par la formule (RO)ₙR'X dans laquelle X représente un atome d'hydrogène, de chlore, de brome ou d'iode et R et R', indépendamment l'un de l'autre, un radical méthyle, éthyle ou propyle, n étant tel que le nombre d'atomes de carbone de l'agent alkylant est ≥ 2, notamment les chlorométhyl polyméthoxy éthers et les chlorométhyl-poly-éthoxy éthers, les oxydes d'éthylène et de propylène et, d'autre part encore, les composés de structure répondant à la formule 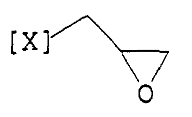 qui représente des épihalohydrines lorsque X représente Cl ou Br, le glycidol lorsque X représente OH, les éthers de glycidyle lorsque X représente OR, R étant une chaîne aliphatique d'au plus 12 atomes de carbone, ou un oxiranne lorsque X est une chaîne aliphatique R de 1 à 12 atomes de carbone.

2. Procédé selon la revendication 1, **caractérisé par le fait que** la proportion de catalyseur par rapport au phénol est de 1 à 50‰, de préférence de 5 à 30‰.

3. Procédé selon l'une des revendications 1 et 2, **caractérisé par le fait que** la quantité de protons présente dans le milieu nitrant est de 2,4 à 6 et préférentiellement de 2,6 à 2,9 équivalents par rapport au phénol et peut être apportée par un acide appelé co-acide et constitué par un acide minéral choisi dans le groupe comprenant les acides chlorhydrique, bromhydrique, sulfurique et phosphorique, ou par un acide organique choisi dans le groupe comprenant les acides carboxyliques inférieurs dont notamment les acides acétique, propionique et oxalique.

4. Procédé selon l'une des revendications 1 à 3, **caractérisé par le fait que** l'agent alkylant mis en oeuvre pendant l'étape d'alkylation est soit un monohalogénure d'alkyle représenté par la formule CₙH₂ₙ₋₁X dans laquelle X est un atome de chlore, brome ou iode et n est ≥ 3, soit un polyhalogénure d'alkyle représenté par la formule formule CₙH₂ₙ₋ₘX₂₊ₘ dans laquelle X est un atome de chlore, brome ou iode et n ≥ 2 avec O ≤ m ≤ 2n.

5. Procédé selon la revendication 4, **caractérisé par le fait que** l'agent alkylant est choisi dans le groupe comprenant,
- en tant que monohalogénures d'alkyle, les halogénures de n-propyle, d'isopropyle, de n-butyle, de sec-butyle, d'isobutyle, de tertbutyle, de n-pentyle, de sec-amyle, de 3-pentyle, de neo-pentyle, d'hexyle, d'isohexyle, de cyclohexyle, de cyclopropyle, de benzyle, d'allyle, et de polyalcoxyalkyle avec comme chaîne alcoxylée un radical méthoxy, éthoxy ou propanoxy,
- en tant que polyhalogénures d'alkyle, les dihaloéthanes, notamment le dichloroéthane et le dibromo-éthane, les dihalopropanes, notamment les 1,2- et 1,3-dichloropropanes.

6. Procédé selon l'une des revendications 1 à 5, **caractérisé par le fait que** l'étape d'amination est réalisée en faisant réagir, sous pression atmosphérique à la température de reflux du milieu, le produit de l'étape d'alkylation avec un excès d'amine secondaire ou primaire choisie dans le groupe comprenant l'amino-3-pentane, la N,N-diéthylamine, la N,N-dipropylamine, la N-éthyl,N-butylamine, la N-propyl,N-méthyl-cyclopropylamine, la N-(2-chloroéthyl), N-propyl- amine, la N-propyl, N-2-méthyl-2-propénylamine, la N-éthyl, N-2-méthyl-2-propénylamine au sein d'un solvant inerte choisi dans le groupe comprenant les alcools et les solvants aromatiques.

## Patentansprüche

1. Verfahren zur Herstellung von 3,4-disubstituierten Dinitroanilinen der Formel 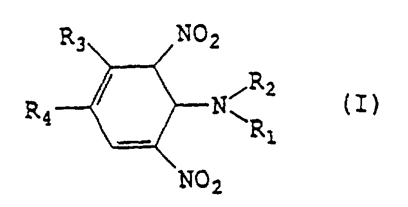 wobei
- R₁ und R₂, die identisch oder voneinander unterschiedlich sind, ein Wasserstoffatom, einen unverzweigten oder verzweigt gesättigten Alkylrest von C₁ bis C₆, einen linearen oder verzweigten Alkylenrest von C₂ bis C₆, einen Cyclopropyl- oder einen Chlorethylrest darstellen,
- R₃ und R₄, die identisch oder voneinander unterschiedlich sein können, aus der Gruppe ausgewählt werden, die das Chloratom, die Aminogruppe, niedere Alkylreste von C₁ bis C₃ und den Trifluormethylrest enthält,
das aufeinanderfolgend die Schritte der Dinitrierung eines 3,4-substituierten Phenols, der Alkylierung des daraus erhaltenen zweifach nitrierten Derivates und der Aminierung des wiederum daraus erhaltenen 3,4-disubstituierten 2,6-Dinitroalkoxybenzols umfasst, und das **gekennzeichnet ist dadurch**, dass:
- der Dinitrierungsschritt des 3,4-disubstituierten Phenols, welches dem gewünschten 3,4-disubstituierten Dinitroanilin entspricht, in einem Reaktionsmilieu durchgeführt wird, das einen Überschuss an Nitrierungsreagenz von 10 bis 30 Gew.-%, eine Menge an Protonen von 2 bis 7 Äquivalenten im Verhältnis zum Phenol und einen Katalysator enthält, der aus der Gruppe bestehend aus den löslichen Salzen der Übergangsmetalle der Gruppen IV bis XII des Periodensystems gewählt wird, vorzugsweise aus den löslichen Salzen der lonen Fe_{III}, Fe_{II}, Zn_{II} und Cu_{II}, und dass
- ein O-Alkylierungsschritt des zweifach nitrierten 3,4-disubstituierten Phenols, das **durch** den vorhergehenden Schritt erhalten wird, mit Hilfe eines Alkylierungsreagenzes durchgeführt wird, das aus der Gruppe gewählt wird, die zum einen lineare oder verzweigte Monohalogenalkylverbindungen mit mindestens 3 Kohlenstoffatomen, wobei ein gesättigter Ring oder mindestens eine ungesättigte Position enthalten sein kann, enthält, und die zum anderen lineare oder verzweigte Polyhalogenalkylverbindungen mit mindestens 2 Kohlenstoffatomen, wobei ein gesättigter Ring oder mindestens eine ungesättigte Position enthalten sein kann, enthält, und des Weiteren Polyalkoxyhaloalkylether, die **durch** die Formel (RO)ₙR'X dargestellt werden, in der X ein Wasserstoff-, Chlor-, Brom- oder Jodatom darstellt und R und R' unabhängig voneinander ein Methyl-, Ethyl- oder Propylrest sein können, und n sowie die Anzahl der Kohlenstoffatome des Alkylierungsreagenzes ≥ 2 ist, insbesondere Chlormethylpolymethoxyether und Chlormethylpolyethoxyether, Ethylenoxide und Propylenoxide und, des Weiteren, Verbindungen der Struktur, die der Formel 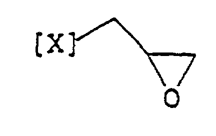 entsprechen, die, wenn X Cl oder Br ist, Epihalohydrine darstellt, wenn X OH ist, Glycidol darstellt, die, wenn X OR ist, wobei R eine aliphatische Kette mit mehr als 12 Kohlenstoffatomen ist, Glycidether darstellt, oder die, wenn X eine aliphatische Kette R mit 1 bis 12 Kohlenstoffatomen ist, ein Oxiran darstellt.

2. Verfahren nach Anspruch 1,
**dadurch gekennzeichnet,**
**dass** der Anteil an Katalysator im Verhältnis zum Phenol von 1 bis 50‰, vorzugsweise von 5 bis 30‰ ist.

3. Verfahren nach einem der Ansprüche 1 und 2,
**dadurch gekennzeichnet,**
**dass** die Menge an im Nitrierungsmilieu enthaltenen Protonen 2,4 bis 6 und bevorzugterweise 2,6 bis 2,9 Äquivalente bezogen auf Phenol ist, und durch eine Säure eingebracht werden kann, die Cosäure genannt wird, bestehend aus einer Mineralsäure, die aus der Gruppe gewählt wird, die Chlorwasserstoff- und Bromwasserstoffsäure, Schwefel- und Phosphorsäure enthält, oder bestehend aus einer organischen Säure, die aus der Gruppe gewählt wird, die die niederen Carbonsäuren enthält, davon insbesondere Essig-, Propion- und Oxalsäure.

4. Verfahren nach einem der Ansprüche 1 bis 3,
**dadurch gekennzeichnet,**
**dass** das Alkylierungsreagenz, das während des Alkylierungsschrittes zugegeben wird, entweder eine Monohalogenalkylverbindung, beschrieben durch die Formel CₙH₂ₙ₋₁X, in der X ein Chlor-, Brom- oder Jodatom und n ≥ 3 ist, oder eine Polyhalogenalkylverbindung ist, die durch die Formel CₙH₂ₙ₋ₘX₂₊ₘ beschrieben wird, in der X ein Chlor-, Brom- oder Jodatom und n ≥ 2 mit 0 ≤ m ≤ 2n ist.

5. Verfahren nach Anspruch 4,
**dadurch gekennzeichnet,**
**dass** das Alkylierungsmittel aus der Gruppe gewählt wird, umfassend,
- in der Eigenschaft als Monohalogenalkylverbindungen, n-Propyl-, Isopropyl-, n-Butyl-, sec-Butyl-, Isobutyl-, tert-Butyl-, n-Pentyl-, sec-Amyl-, 3-Pentyl-, neo-Pentyl-, Hexyl-, Isohexyl-, Cyclohexyl-, Cyclopropyl-, Benzyl-, Allylhalogenid und Polyalkoxyalkylhalogenid, wobei die Alkoxykette ein Methoxy-, Ethoxy- oder Propoxyrest ist,
- in der Eigenschaft als Polyhalogenalkylverbindungen, Dihalogenethane,insbesondere Dichlorethan und Dibromethan, die Dihalopropane, insbesondere 1,2- und 1,3-Dichlorpropan.

6. Verfahren nach einem der Ansprüche 1 bis 5,
**dadurch gekennzeichnet,**
**dass** der Aminierungsschritt durchgeführt wird, indem man bei Atmosphärendruck unter Rückflusstemperatur des Milieus das Produkt des Alkylierungsschrittes mit einem Überschuss an sekundärem oder primärem Amin, das aus der Gruppe gewählt wird, die Amino-3-pentan, N,N-Diethylamin, N,N-Dipropylamin, N-Ethyl- N-butylamin, N-Propyl- N-methylcyclopropylamin, N-(2-Chlorethyl)- N-propylamin, N-Propyl- N-2-methyl-2-propenylamin, N-Ethyl- N-2-methyl-2-propenylamin enthält, in einem inerten Lösungsmittel, das aus der Gruppe ausgewählt wird, die Alkohole und aromatische Lösungsmittel enthält, zur Reaktion bringt.

## Claims

1. Preparation process for 3,4-disubstituted dinitroanilines of formula 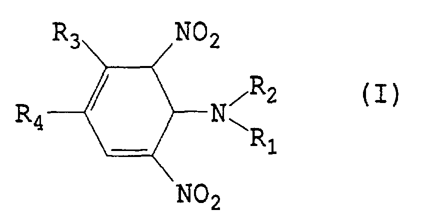 in which
- R1 and R2, which are identical to or different from one another, represent a hydrogen atom, a C1 to C6 saturated, linear or branched alkyl radical, a C2 to C6
linear or branched alkylene radical, a cyclopropyl radical or a chloroethyl radical,
- R3 and R 4 which are identical to or different from one another, are chosen from the group containing the chlorine atom, the amino group, the C1 to C3 lower alkyl radicals and the trifluoromethyl radical, comprising successively, a dinitration stage of a 3,4disubstituted phenol, an alkylation stage of the dinitrated derivative thus obtained and an amination stage of the 3,4 disubstituted 2,6-dinitro- alkoxybenzene thus obtained and
**characterized in that**:
- the dinitration stage of the 3,4-disubstituted phenol corresponding to the sought 3,4-disubstituted dinitroaniline is carried out in a reaction medium containing a slight excess of nitrating agent, a sufficient quantity of protons and a catalyst chosen from the group containing the soluble salts of the transition metals of columns IV to XII of the Periodic Table, preferably the soluble salts of Fe_{III}, Fe_{II}, Zn_{II} and Cu_{II}ions and that
- an 0-alkylation stage of the dinitrated 3,4disubstituted phenol obtained in the preceding stage, is 35 carried out using an alkylating agent chosen from the group containing, on the one hand, linear or branched alkyl mono-halides having at least 3 carbon atoms and which can contain a saturated ring or at least one unsaturation, on the other hand, linear or branched alkyl polyhalides having at least 5 2 carbon atoms and which can contain a saturated ring or at least one unsaturation, or yet again, polyalkoxy-haloalkyl ethers represented by the formula (RO)nR'X in which X represents a hydrogen, chlorine, bromine or iodine atom and R and R', independently of one another represent a methyl, ethyl or propyl radical, n being such that the number of carbon atoms of the alkylating agent is >_ 2, in particular chloromethyl polymethoxy ethers and chloromethyl-polyethoxy ethers, the oxides of ethylene and propylene and, yet again, the compounds with a structure corresponding to the formula 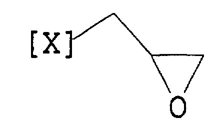 which represents epihalohydrins when X represents Cl or Br, glycidol when X represents OH, glycidyl ethers when X represents OR, R being an aliphatic chain with at most 12 carbon atoms, or an oxirane when X is an aliphatic chain R with 1 to 12 carbon atoms.

2. Process according to claim 1, **characterized in that** the proportion of catalyst relative to phenol is 1 to 50°/oo, preferably 5 to 30°/oo.

3. Process according to one of claims 1 and 2, **characterized in that** the quantity of protons present in the nitrating medium is 2 to 7, preferably 2.4 to 6 and more preferably still, 2.6 to 2.9 equivalents relative to the phenol and can be provided by an acid called a co-acid and constituted by an inorganic acid chosen from the group containing hydrochloric, hydrobromic, sulphuric and phosphoric acids, or by an organic acid chosen from the group containing lower carboxylic acids in particular acetic, propionic and oxalic acids.

4. Process according to one of claims 1 to 3, **characterized in that** the alkylating agent used during the alkylation stage is either an alkyl monohalide represented by the formula CnH2n-1X in which X is a chlorine, bromine or iodine atom and n is s 3, or an alkyl polyhalide represented by the formula CnH2n-mX2+m in which X is a chlorine, bromine or iodine atom and n is >_ 2 with 0 <_ m <_ 2n.

5. Process according to claim 4, **characterized in that** the alkylating agent is chosen from the group containing,
- as alkyl monohalides, n-propyl, isopropyl, n-butyl, sec-butyl, isobutyl, tertbutyl, n-pentyl, sec-amyl, 3-pentyl, neopentyl, hexyl, isohexyl, cyclohexyl, cyclopropyl, benzyl, allyl halides and polyalkoxyalkyl halides with a methoxy, ethoxy or propanoxy radical as the alkoxylated chain,
- as alkyl polyhalides, the dihaloethanes, in particular dichloroethane and dibromoethane, the dihalo propanes, in particular 1,2- and 1,3-dichloropropanes.

6. Process according to one of claims 1 to 5, **characterized in that** the amination stage is carried out by reacting, under atmospheric pressure and at reflux temperature of the medium, the product of the alkylation stage with an excess of secondary or primary amine chosen from the group containing amino-3-pentane, N,N-diethylamine, N,N-dipropylamine, N-ethyl-, N-butylamine, N-propyl,N-methyl cyclopropylamine, N-(2-chloroethyl), N-propylamine, N-propyl, N-2-methyl-2-propenylamine, N-ethyl,N-2-methyl-2 propenylamine in an inert solvent chosen from the group containing alcohols and aromatic solvents.
